# EUROPÄISCHE PATENTANMELDUNG

(11) **EP 4 736 903 A1**
(43) Veröffentlichungstag der Anmeldung: **06.05.2026**
(21) Anmeldenummer: 24210275.4
(22) Anmeldetag: 31.10.2024
(51) Int. Cl.: A61M 1/06

(54) **MILCHPUMPENSYSTEM**

(71) Anmelder: MAM Baby AG, 8832 Wollerau (CH)
(72) Erfinder: ROHACZEK, Thomas, 1210 Wien (AT)
(74) Vertreter: SONN Patentanwälte GmbH & Co KG

(57) **Zusammenfassung**

Milchpumpensystem (1) aufweisend
- ein Verbindungselement (7) zur Verbindung eines Milchbehälters (2) mit einer Brusthaube (4), wobei das Verbindungselement (7) einen Kanal (8) zur Verbindung eines Hohlraums (3) des Milchbehälters (2) mit einem Nippeltunnel (6) der Brusthaube (4) aufweist;
- ein Pumpelement (10), das dazu eingerichtet ist, einen Unterdruck im Kanal (8) des Verbindungselements (7) zu erzeugen;
- einen Handgriff (11), der dazu eingerichtet ist, das Pumpelement (10) zu betätigen;
wobei das Verbindungselement (7) ein Führungselement (12) in Form einer Nut oder länglich verlaufenden Auskragung (14) aufweist und der Handgriff (11) ein Halteelement (15) aufweist, wobei das Halteelement (15) und das Führungselement (12) miteinander in Eingriff stehen, sodass der Handgriff (11) um die Eingriffsstelle (16) zum Betätigen des Pumpelements (10) schwenkbar ist.

## Beschreibung

Die Erfindung betrifft ein Milchpumpensystem (Brustpumpensystem) aufweisend:
- ein Verbindungselement zur Verbindung eines Milchbehälters mit einer Brusthaube, wobei das Verbindungselement einen Kanal zur (insbesondere fluidischen) Verbindung eines Hohlraums des Milchbehälters mit einem Nippeltunnel der Brusthaube aufweist;
- ein Pumpelement, das dazu eingerichtet ist, einen Unterdruck im Kanal des Verbindungselements zu erzeugen;
- einen Handgriff, der dazu eingerichtet ist, das Pumpelement zu betätigen.

Es ist bekannt, bei Brustpumpen die Position des Handgriffs veränderbar zu gestalten. So zeigt EP 3795188 B1 eine Handmilchpumpe aufweisend: einen Hauptkörper mit einem Durchlass, durch den abgepumpte Brustmilch hindurchtritt; eine Haube, die mit dem Hauptkörper verbunden und auf einer Brust platziert ist; eine Membran, die für den Hauptkörper bereitgestellt ist und im Durchlass einen Unterdruck erzeugt. Außerdem ist ein Halteelement vorgesehen, das auf dem Hauptkörper befestigt und bereitgestellt ist, um in Bezug auf den Hauptkörper drehbar zu sein und ein Griff, mit dem die Membran verformt werden kann, wobei der Griff durch das Halteelement gehalten wird. Der Griff und das Halteelement können dabei gemeinsam in Bezug auf den Hauptkörper gedreht werden. Dabei ist der Griff über einen Spindelabschnitt mit dem Halteelement verbunden. Nachteiligerweise ist damit der Zusammenbau der Brustpumpe kompliziert, da einerseits das Halteelement am Hauptkörper und andererseits der Griff am Spindelabschnitt befestigt werden müssen. Andererseits erfolgt beim gleichzeitigen Verdrehen des Griffs und des Halteelements die Kraftübertragung über den Spindelabschnitt, wobei es zu einem Verkippen des Griffs relativ zum Halteelement oder zu einem Verkanten des Halteelements am Hauptkörper kommen kann. Aufgrund des Halteelements ist der Schwenkpunkt des Griffs zum Pumpen außerdem weit nach oben (noch oberhalb der Membran) verlagert, worunter die Ergonomie beim Pumpen leiden kann und ein großer Hub beim Pumpen notwendig ist. Eine ähnliche Brustpumpe zeigt EP 3646902 B1, die mit ähnlichen Nachteilen verbunden ist.

Eine weitere ähnliche Brustpumpe, die auch eine Schwenkachse verwendet, zeigt EP 4032566 B1. Als zusätzlicher Nachteil ist dabei der Handgriff nicht um die Symmetrieachse des Milchbehälters drehbar, sondern um eine demgegenüber verkippte Achse.

In US 7,727,182 B2 ist der Handgriff zwar drehbar, jedoch ebenfalls nicht um eine Symmetrieachse des Milchbehälters. Außerdem stützt sich der Handgriff beim Pumpen nur an der flexiblen Membran ab, was zu einer schlechten Pumpleistung führen kann.

Bei diesen Brustpumpen gestaltet sich auch der Zusammenbau schwierig.

Es ist eine Aufgabe der vorliegenden Erfindung, zumindest einen der Nachteile des Stands der Technik zu lindern oder zu beheben. Insbesondere ist es eine Aufgabe der vorliegenden Erfindung, eine Brustpumpe mit einem schwenkbaren Handgriff vorzuschlagen, die einfach zusammenzubauen ist, die einen geringen Hub des Handgriffs zum Pumpen erfordert, bei der der Handgriff in einer beliebigen Stellung im Schwenkbereich montierbar ist, bei der kein Demontieren und Wiedermontieren für die Winkelverstellung notwendig ist, bei der in jeder Schwenkstellung im Wesentlichen derselbe Maximalhub erforderlich ist und/oder derselbe Maximalunterdruck erzeugt wird, und/oder die möglichst wenige separate Bauteile aufweist.

Dies wird gelöst durch ein Milchpumpensystem wie eingangs angeführt, wobei das Verbindungselement ein Führungselement in Form einer Nut oder länglich verlaufenden Auskragung aufweist und der Handgriff ein Halteelement aufweist, wobei das Halteelement und das Führungselement miteinander in Eingriff stehen, sodass der Handgriff um die Eingriffsstelle zum Betätigen des Pumpelements schwenkbar ist.

Der Handgriff ist somit direkt am Verbindungselement abgestützt. Dadurch entfällt die Notwendigkeit eines Zwischenstücks zwischen Handgriff und Verbindungselement. Andererseits ist somit der Schwenkpunkt nahe am Verbindungselement, wodurch der beim Pumpen notwendige Hub bzw. der beim Pumpen mit der Hand zu umfassende Abstand gering ist und somit das Pumpen erleichtert wird bzw. auch Personen mit kleinen Händen ein ergonomisches Pumpen ermöglicht wird. Da das Führungselement durch eine länglich verlaufende Auskragung bzw. eine (länglich verlaufende) Nut gebildet ist, kann das Halteelement insbesondere entlang des Führungselements (insbesondere in Umfangsrichtung) verschoben werden. Der Handgriff ist somit insbesondere verdrehbar. Somit kann die Stellung des Handgriffs verändert werden und somit eine ergonomischere Stellung erzielt werden. Die Brustpumpe kann insbesondere in jeder Drehstellung des Handgriffs zusammengebaut werden, indem das Halteelement an der betreffenden Stelle mit dem Führungselement in Eingriff gebracht wird. Darunter, dass das Halteelement und das Führungselement miteinander in Eingriff stehen wird insbesondere verstanden, dass das Halteelement in das Führungselement eingreift und/oder dass das Führungselement in das Halteelement eingreift.

Das Milchpumpensystem ist vorzugsweise eine Milchpumpe oder ein Element einer Milchpumpe (bspw. verbindbar mit einer (externen) Brusthaube und/oder einem (externen) Milchbehälter). Das Milchpumpensystem muss den Milchbehälter und/oder die Brusthaube nicht aufweisen, kann jedoch den Milchbehälter und/oder die Brusthaube aufweisen. Vorzugsweise ist das Verbindungselement zur lösbaren Verbindung (bspw. durch Anschrauben) mit einem Milchbehälter ausgebildet. Alternativ kann das Verbindungselement auch einstückig mit dem Milchbehälter ausgeführt sein. Vorzugsweise ist das Verbindungselement zur lösbaren Verbindung mit einer Brusthaube ausgebildet, insbesondere durch Einstecken des Nippeltunnels der Brusthaube in das Verbindungselement. Alternativ kann das Verbindungselement auch einstückig mit der Brusthaube ausgeführt sein. Vorzugsweise ist der Kanal des Verbindungselements zur Verbindung einer Öffnung zum Hohlraum des Milchbehälters mit dem Nippeltunnel der Brusthaube.

Das Halteelement ist vorzugsweise in Form eines Haltevorsprungs und/oder einer Halteeinbuchtung (also insbesondere einen konvex verlaufenden Abschnitt) gebildet. Es kann auch ein Haltevorsprung vorgesehen sein, der eine Einbuchtung aufweist, mit der das Führungselement in Eingriff steht. Das Halteelement und das Führungselement sind insbesondere derart ausgebildet, dass der Handgriff über das Halteelement am Führungselement beim Pumpen abgestützt ist und die Eingriffstelle zwischen dem Halteelement und dem Führungselement eine Schwenkachse definiert, um die beim

Pumpen geschwenkt wird.

Vorzugsweise verläuft eine Schwenkachse des Handgriffs (zum Pumpen, d.h. zum Betätigen des Pumpelements durch den Benutzer) durch die Eingriffsstelle. Vorzugsweise weist der Handgriff einen Halteabschnitt zur Verbindung mit dem Pumpelement und einen Hebelarm (zur Betätigung durch einen Benutzer der Milchpumpe bzw. des Milchpumpensystems) auf. Vorzugsweise verläuft die Schwenkachse im Wesentlichen senkrecht zur Längserstreckungsrichtung des Hebelarms. Vorzugsweise sind der Halteabschnitt und der Hebelarm winkelig miteinander verbunden. Vorzugsweise sind zwei Halteelemente vorgesehen, wobei einerseits jedes der Halteelemente jeweils und andererseits das Führungselement miteinander in Eingriff stehen. Die Halteelemente sind vorzugsweise parallel zueinander ausgebildet und/oder in der gleichen Form ausgebildet. Alternativ können die Halteelemente in einem spitzen oder stumpfen Winkel zueinander stehen. Die Schwenkachse des Handgriffs (zum Betätigen des Pumpelements) wird in dieser Ausführung somit durch die zwei Eingriffsstellen definiert. Vorzugsweise weist jedes der Halteelemente eines oder mehrere der in dieser Offenbarung beschriebenen bevorzugten Ausgestaltungen auf.

Vorzugsweise weist das Pumpelement eine Membran auf, über die das Pumpenelement (insbesondere in Verbindung mit dem unten erwähnten Rückschlagventil im Kanal) über eine Öffnung im Verbindungselement, insbesondere im Kanal des Verbindungselements, einen Unterdruck im Verbindungselement bzw. im Milchflusskanal erzeugen kann. Vorzugsweise ist das Pumpelement einstückig ausgeführt. Vorzugsweise ist der Handgriff einstückig ausgeführt. Vorzugsweise ist das Verbindungselement einstückig ausgeführt. Vorzugsweise ist das Milchpumpensystem eine manuelle Milchpumpe oder geeignet zur Verwendung in einer manuellen Milchpumpe. Alternativ ist das Milchpumpensystem eine elektrische Milchpumpe oder geeignet zur Verwendung in einer elektrischen Milchpumpe. Vorzugsweise weist das Pumpelement ein elastisches Material, mit einer Shore-A-Härte von bevorzugt zwischen 0 und 100, besonders bevorzugt zwischen 20 und 85, noch mehr bevorzugt zwischen 40 und 70, auf. Vorzugsweise besteht das Pumpelement aus einem elastischen Material. Das Pumpelement kann unterschiedliche Materialien aufweisen. Das Pumpelement kann bspw. mittels Mehrkomponenten-Spritzgießen hergestellt sein.

Die Nut ist insbesondere eine sich länglich erstreckende Vertiefung. Die Auskragung ist insbesondere ein sich länglich erstreckender Vorsprung. Als Nut kann bspw. eine Rille vorgesehen sein. Vorzugsweise weist das Führungselement in einem Querschnitt senkrecht zu einer Längserstreckungsrichtung des Führungselement zumindest in einem Abschnitt entlang der Längserstreckungsrichtung einen unveränderlichen Querschnitt auf (insbesondere in einem Verschiebeabschnitt, entlang dessen das Halteelement verschiebbar ist; wobei der Verschiebeabschnitt vorzugsweise zumindest 180° umfasst). Vorzugsweise verläuft das Führungselement rotationssymmetrisch zu einer Pumpenhauptachse.

Das Verbindungselement weist vorzugsweise einen Brusthauben-Verbindungsabschnitt auf, der zur Verbindung mit dem Nippeltunnel vorgesehen ist bzw. der den Nippeltunnel aufnimmt. Der Brusthauben-Verbindungsabschnitt ist insbesondere allgemein zylinderförmig oder konisch ausgebildet. Die Brusthaube ist vorzugsweise mit dem Nippeltunnel in das Verbindungselement einsteckbar und wird in diesem durch Reibschluss gehalten. Vorzugsweise weist der Nippeltunnel im aus dem Brusthauben-Verbindungsabschnitt entfernten Zustand einen Außenumfang auf, der gleich oder größer als ein Innenumfang des Brusthauben-Verbindungsabschnitts ist. Durch die Übermäßigkeit des Nippeltunnels ist dieser vorteilhafterweise zum Verbindungselement dichtend. Vorzugsweise ist die Brusthaube vom Verbindungselement zerstörungsfrei trennbar. Der Brusthauben-Verbindungsabschnitt hat vorzugsweise die Form eines allgemeinen Zylinders, insbesondere eines Kreiszylinders oder eine elliptische Ausformung. Der Nippeltunnel hat vorzugsweise die Form eines allgemeinen Zylinders oder eines Kegelstumpfes (Konus). Der Brusthauben-Verbindungsabschnitt des Verbindungselements ist vorzugsweise an den Nippeltunnel angepasst. Statt der allgemein zylindrischen Form kann auch eine kegelstumpfförmige oder pyramidenstumpfförmige Form für den Nippeltunnel und/oder den Brusthauben-Verbindungsabschnitt vorgesehen sein. Weiters weist das Verbindungselement vorzugsweise ein Anschlusselement zur Verbindung mit dem (externen oder vom Milchpumpensystem umfassten) Milchbehälter auf. Das Anschlusselement ist bevorzugt allgemein zylindrisch. Der Milchbehälter ist vorzugsweise mit dem Verbindungselement verbindbar, z.B. am Verbindungselement anschraubbar, ansteckbar oder über einen Bajonettverschluss verbindbar. Vorzugsweise weist das Anschlusselement eine axiale Erstreckungsrichtung auf, die in einem Winkel zwischen 65° und 155°, weiter bevorzugt zwischen 80° und 140°, besonders bevorzugt im Wesentlichen senkrecht, zu einer axialen Erstreckungsrichtung des Brusthauben-Verbindungsabschnitts steht. Darunter, dass der Brusthauben-Verbindungsabschnitt bzw. der Nippeltunnel insbesondere allgemein zylinderförmig oder konisch ausgebildet sind, wird insbesondere verstanden, dass diese die Form der Mantelfläche eines allgemeinen Zylinders bzw. Konus (d.h. Kegelstumpfes) aufweisen.

Der Brusttrichter ist insbesondere zur (teilweisen) Aufnahme einer Brust eingerichtet. Der Brusttrichter kann fest oder lösbar mit dem Nippeltunnel verbunden sein. Der Brusttrichter ist optional somit austauschbar. Der Brusttrichter kann bspw. gerade oder geknickt ausgeführt sein. Der (externe oder vom Milchpumpensystem umfasste) Milchbehälter ist vorzugsweise starr ausgebildet. Vorzugweise bildet der Kanal einen Milchflussweg vom Nippeltunnel bzw. von der Brusthaube zum Hohlraum des Milchbehälters, wobei der Milchflussweg insbesondere durch ein Ventil verläuft, das vorzugsweise den Vakuumaufbau im Kanal bzw. im Nippeltunnel ermöglicht, bspw. ein Rückschlagventil (Ein-Wege-Ventil), insbesondere ein Lippen-Rückschlagventil (duckbill valve). Insbesondere ist im Kanal, in der Öffnung zum Hohlraum des Milchbehälters, das Rückschlagventil vorgesehen. Vorzugsweise ist das Rückschlagventil einstückig ausgebildet. Optional weist der Kanal einen Nippeltunnel-Abschnitt auf, der zum Nippeltunnel führt, und einen Milchbehälter-Abschnitt, der zum Hohlraum des Milchbehälters führt, wobei die beiden Abschnitte vorzugsweise winkelig miteinander verbunden sind. Optional setzt das Rückschlagventil in einer Hälfte des Milchbehälter-Abschnitts an, der näher beim Pumpelement liegt. Damit kann der zu evakuierende Raum weiter verkleinert werden. Optional liegt das Rückschlagventil außerhalb des vom Milchbehälter begrenzten Raums. Beim Abpumpen wird die abgepumpte Milch entlang des Milchflusswegs geführt. Vorzugsweise weist der Kanal eine Öffnung bzw. eine Verbindung auf, über die das Pumpenelement einen Unterdruck im Verbindungselement bzw. im Milchflusskanal erzeugen kann.

Es ist vorteilhaft, wenn das Halteelement entlang des Führungselements verschiebbar ist.

Es ist bevorzugt, wenn sich Führungselement im Wesentlichen entlang eines Kreisbogens oder eines (offenen oder geschlossenen) Polygonzugs erstreckt. Vorzugsweise liegt eine Drehachse des Handgriffs im Mittelpunkt des Kreisbogens. Somit ändert sich der Hubwinkel des Handgriffs beim Verdrehen nicht.

Es ist vorteilhaft, wenn das Führungselement in Umfangsrichtung des Verbindungselement über einen Winkelbereich von zumindest 30°, bevorzugt zumindest 90°, besonders bevorzugt zumindest 180°, noch mehr bevorzugt zumindest 250°, noch weiter bevorzugt zumindest 270°, verläuft. Zumindest kann der Handgriff über einen großen Winkelbereich eingestellt werden. Insbesondere kann somit eine linkshändige und eine rechtshändige Einstellung ermöglicht werden. Außerdem wird ein leichteres Abpumpen durch eine weitere Person (z.B. Hebamme) ermöglicht.

Es ist vorteilhaft, wenn der Handgriff durch Verschieben des Halteelements entlang des Führungselements drehbar um eine (insbesondere die oben erwähnte) Drehachse ist. Vorzugsweise ist der maximale Schwenkwinkel, um den der Handgriff zum Betätigen des Pumpelements schwenkbar ist, unabhängig von der Drehstellung des Handgriffs um die Drehachse.

Es ist bevorzugt, wenn das Verbindungselement einen (Milchbehälter-)Verbindungsabschnitt zur Befestigung des (insbesondere externen oder vom Milchpumpensystem umfassten) Milchbehälters aufweist, wobei der Verbindungsabschnitt im Wesentlichen eine rotationssymmetrische Mantelfläche aufweist, wobei die Drehachse des Handgriffs parallel zu einer Rotationssymmetrieachse der Mantelfläche ist, wobei insbesondere die Drehachse und die Rotationssymmetrieachse zusammenfallen. Dadurch ergibt sich, dass der Handgriff in jeder Dreh (winkel)stellung den gleichen Schwenkhub und damit den gleichen Vakuumwert ermöglicht. Außerdem ist damit der beim Pumpen mit der Hand zu umfassende Abstand unabhängig von der gewählten Drehstellung. Der Milchbehälter-Verbindungsabschnitt weist vorzugsweise ein Gewinde auf.

Es ist bevorzugt, wenn der Milchbehälter eine im Wesentlichen rotationssymmetrische Mantelfläche aufweist, wobei die Drehachse des Handgriffs parallel zu einer Rotationssymmetrieachse der Mantelfläche des Milchbehälters ist, wobei diese insbesondere zusammenfallen.

Es ist vorteilhaft, wenn das Halteelement (insbesondere in der Ausformung als Haltevorsprung) ein gabelförmiges Endstück aufweist, dass mit dem Führungselement in Eingriff steht. Vorzugsweise ist das Führungselement dabei als Auskragung ausgeführt. Dadurch ist das Führungselement besonders gut herstellbar. Insbesondere kann somit das Führungselement in das Halteelement eingreifen bzw. (teilweise) in diesem liegen. Insbesondere weist das Halteelement auf seinem (dem Hebelarm abgewandten) Ende zwei Arme auf.

Es ist bevorzugt, wenn das Pumpelement einen Haltezapfen und der Handgriff eine Halteöffnung aufweist, wobei der Haltezapfen durch die Halteöffnung geführt ist und eine Hinterschneidung aufweist, um den Handgriff (insbesondere mittels Schnappverbindung) zu halten, sodass ein Verschwenken des Handgriffs um die Eingriffsstelle ein Betätigen des Pumpelements bewirkt. Ein Verschwenken des Handgriffs (insbesondere des Hebelarms) in Richtung des Verbindungselements bzw. des Milchbehälters führt somit zu einem Zug am Haltezapfen des Pumpelements weg vom Verbindungselements (d.h. nach oben in einer Standstellung der Milchpumpe bzw. des Milchpumpensystems mit einem verbundenen (externen) Milchbehälter). Vorzugsweise weist die Hinterschneidung eine Vorspannung des Haltezapfens und/oder der Handgriff im Bereich der Halteöffnung im auseinandergenommenen (demontierten) Zustand eine Vorspannung auf, sodass im zusammengebauten (montierten) Zustand der Handgriff von der Hinterschneidung in Richtung des Verbindungselements (bzw. in Richtung einer Pumpmembran des Pumpelements) gezogen wird. Insbesondere verläuft eine Drehachse des Handgriffs durch den Haltezapfen und/oder ist im Mittelpunkt der Halteöffnung. Der Handgriff ist somit leicht verdrehbar, wobei beim Verdrehen der Handgriff mit der Halteöffnung um den Haltezapfen rotiert (und das Halteelement entlang des Führungselements geführt wird). Zum Montieren wird der Haltezapfen mit der Hinterschneidung durch die Halteöffnung gezogen/gedrückt. Vorzugsweise ist der Haltezapfen mit der Hinterschneidung zerstörungsfrei durch die Halteöffnung ziehbar und/oder drückbar. Vorzugsweise ist eine dem Milchbehälter abgewandte Oberseite des Haltezapfens geschlossen.

Es ist vorteilhaft, wenn das Verbindungselement einen (insbesondere schalenförmigen) Aufnahmeraum für das Pumpelement ausbildet, der mit dem Kanal verbunden ist, wobei das Pumpelement den Aufnahmeraum im Wesentlichen abdeckt. Beim Betätigen des Pumpelements wird der Raum zwischen Pumpelement und Aufnahmeraum vergrößert, um einen Unterdruck zu erzeugen. Eine Schalenform erlaubt insbesondere gegenüber einer Topfform einen geringeren notwendigen Hub zum Erzeugen eines vorbestimmten Unterdrucks und verbessert die Reinigbarkeit.

Es ist vorteilhaft, wenn das Pumpelement (insbesondere angrenzend an den schalenförmigen Abschnitt) einen umlaufenden Schnapprand aufweist, der mit einem den Aufnahmeraum begrenzenden umlaufenden Befestigungsrand des Verbindungselements in Schnappverbindung steht. Damit wird das Schnappelement am Verbindungselement gehalten. Zum Zusammenbauen des Milchpumpensystems wird insbesondere der Schnapprand über den Befestigungsrand geschoben/gezogen/gedrückt. Vorzugsweise sind der Schnapprand und der Befestigungsrand derart ausgeführt, dass sich das Pumpelement beim Pumpen an dem Verbindungselement ansaugt. Dies wirkt insbesondere der mechanischen Abzugskraft beim Pumpen entgegen. Vorzugsweise dichtet der Schnapprand den Aufnahmeraum ab.

Vorzugsweise weist das Pumpelement eine insbesondere umfänglich verlaufende Dichtlippe auf. Die Dichtlippe verläuft vorzugsweise (umfänglich) auf einem umfänglichen Flansch des Pumpelements, der an jenen Abschnitt des Pumpelements anschließt bzw. jenen Abschnitt des Pumpelements begrenzt, der den Aufnahmeraum abdeckt oder im Aufnahmeraum liegt. Vorzugsweise schließt der Schnapprand (insbesondere umfänglich) an den umfänglichen Flansch an. Mit der Dichtlippe kann vorteilhafterweise dasselbe Pumpelement zum manuellen und zum elektrischen Pumpen verwendet werden.

Vorzugsweise ist der Aufnahmeraum (bis auf einen Bereich um eine Öffnung für eine Verbindung zum Kanal des Verbindungselements, der die Öffnung zum Hohlraum des Milchbehälters mit dem Nippeltunnel verbindet, wobei über die Verbindung mit dem Pumpelement ein Unterdruck im Kanal erzeugbar ist) kugelkalottenförmig (kugelkappenförmig). Kugelkalotte bezeichnet dabei den gekrümmten Teil der Oberfläche eines Kugelsegments. Ein Kugelsegment ist der Teil eines Kugelkörpers, der durch den Schnitt mit einer Ebene abgetrennt wird. Im Bereich um die Öffnung für die Verbindung zum Kanal ist der Aufnahmeraum optional flach. Der Bereich weist vorzugsweise einen Radius von weniger als 15 mm, bevorzugt weniger als 1 mm, besonders bevorzugt weniger als 0,7 mm auf. Alternativ kann der Aufnahmeraum bspw. im Querschnitt eine elliptische Form, eine Wellenstruktur oder variable Radienverläufe aufweisen. Der Aufnahmeraum kann auch zylinderförmig, kegelförmig, kegelstumpfförmig, topfförmig oder flach ausgebildet sein.

Insbesondere ist der Aufnahmeraum rotationsymmetrisch ausgebildet. Insbesondere weist das Verbindungselement im Bereich des schalenförmigen Aufnahmeraums eine Krümmung mit einem Krümmungsradius von zwischen 10 mm und 65 mm, bevorzugt zwischen 15 mm und 45 mm, besonders bevorzugt zwischen 20 mm und 30 mm, (in einem Schnitt durch eine Ebene, in der eine Rotationssymmetrieachse des Aufnahmeraums liegt) auf. Insbesondere variiert der Krümmungsradius des Verbindungselements im Bereich des schalenförmigen Aufnahmeraums um weniger als 50%, bevorzugt weniger als 30%, in Bezug auf einen maximalen Krümmungsradius (in einem Schnitt durch eine Ebene, in der eine Rotationssymmetrieachse des Aufnahmeraums liegt). Eine Wand des Verbindungselement in einem an den umlaufenden Rand des Aufnahmeraums anschließenden umlaufenden Randbereich innerhalb des Aufnahmeraums schließt mit der Rotationssymmetrieachse des Aufnahmeraums einen Winkel von bevorzugt weniger als 30°, besonders bevorzugt weniger als 20°, noch mehr bevorzugt weniger als 10°, ein. Dieser steile bis senkrechte innere Rand des Verbindungselements unterstützt einen dichten und stabilen Sitz beim Pumpen.

Es ist bevorzugt, wenn das Pumpelement (in einer Ruhestellung des Handgriffs) am Verbindungselement im Bereich des Aufnahmeraums zumindest abschnittsweise anliegend oder im Wesentlichen anliegend ausgeführt ist. Damit wird die Größe des zu evakuierenden Raums möglichst klein gehalten.

Es ist vorteilhaft, wenn das Pumpelement einen (insbesondere schalenförmigen) Abschnitt aufweist, wobei durch eine Verformung des schalenförmigen Abschnitts der Unterdruck im Kanal des Verbindungselements erzeugbar ist.

Es ist vorteilhaft, wenn eine dem Verbindungselement zugewandte Außenfläche des Pumpelements im schalenförmigen Abschnitt im Wesentlichen rotationssymmetrisch ist. Damit wird eine 360° freie Montage des Pumpelements am Verbindungselement ermöglicht (insbesondere wenn der Aufnahmeraum vom Verbindungselement ebenfalls rotationssymmetrisch ausgebildet wird). Vorzugsweise ist eine dem Pumpelement zugewandte Außenfläche des Verbindungselements im Aufnahmeraum rotationsymmetrisch. Die dem Verbindungselement zugewandte Außenfläche des Pumpelements schließt in einem an den Schnapprand anschließenden umlaufenden Randbereich mit der Rotationssymmetrieachse der Außenfläche einen Winkel von bevorzugt weniger als 30°, besonders bevorzugt weniger als 20°, noch mehr bevorzugt weniger als 10°, ein. Dieser steile bis senkrechte innere Rand des Pumpelements unterstützt einen dichten und stabilen Sitz beim Pumpen.

Es ist bevorzugt, wenn der schalenförmige Abschnitt des Pumpelements eine Tiefe aufweist, gemessen von einer Spitze des schalenförmigen Abschnitts zu einer Ebene, in der ein Rand des schalenförmigen Abschnitts (insbesondere eine Linie, an der der schalenförmige Abschnitt vom Schnapprand begrenzt wird) liegt, und der schalenförmige Abschnitt eine Breite aufweist, die der größten Erstreckung in der Ebene, in der der Rand des schalenförmigen Abschnitts liegt, entspricht, wobei die Breite mindestens 1,5-mal, bevorzugt zumindest doppelt, besonders bevorzugt 2,5-mal, so groß wie die Tiefe ist. Eine flache Geometrie ermöglicht geringere notwendige Hubweiten.

Vorzugsweise weist das Pumpelement auf einer dem Verbindungselement abgewandten Seite Versteifungsrippen auf. Diese ermöglichen ein höheres Vakuum bei kurzem Hub, und damit eine ergonomischere Gestaltung des Milchpumpensystems. Außerdem ermöglichen die Rippen eine Rückstellung des Handgriffs in eine Ruhestellung, auch wenn kein Unterdruck im Kanal vorliegt.

Es ist vorteilhaft, wenn der Handgriff ein in Längsrichtung des Handgriffs verschiebbares (insbesondere am Hebelarm in Längsrichtung des Hebelarms verschiebbares) Anschlagelement aufweist, das in zumindest einer Schiebestellung beim Verschwenken des Handgriffs zur Betätigung des Pumpelements am Verbindungselement anschlägt, sodass das Anschlagelement in der zumindest einen Schiebestellung einen Schwenkwinkel des Handgriffs begrenzt. Vorzugsweise begrenzt das Anschlagelement in einer zweiten Schiebestellung den Schwenkwinkel auf einen anderen Winkel. Vorzugsweise wird der maximale Schwenkwinkel des Handgriffs begrenzt durch ein Anschlagen des Handgriffs an dem Verbindungselement oder dem Milchbehälter. Bevorzugt begrenzt das Anschlagelement in einer weiteren (z.B. dritten) Schiebestellung den Schwenkwinkel nicht, sodass der maximale Schwenkwinkel des Handgriffs durch ein Anschlagen des Handgriffs am Verbindungselement oder am Milchbehälter vorgegeben ist.

Vorzugsweise weist das Verbindungselement einen Abschnitt mit zunehmendem Querschnitt auf, wobei das Anschlagelement in Längsrichtung des Handgriffs zumindest teilweise entlang des Abschnitts mit zunehmendem Außenquerschnitt verschiebbar ist, sodass das Anschlagelement beim Verschwenken des Handgriffs in einer ersten Schiebestellung an ersten Stelle des Abschnitts des Verbindungselements mit zunehmenden Außenquerschnitt anschlägt und in einer zweiten Schiebestellung an einer zweiten Stelle des Abschnitts des Verbindungselements mit zunehmendem Querschnitt anschlägt, sodass das Anschlagelement in der ersten Schiebestellung den Schwenkwinkel des Handgriffs auf einen ersten Winkel begrenzt und in der zweiten Schiebestellung den Schwenkwinkel des Handgriffs auf einen zweiten (vom ersten verschiedenen) Winkel begrenzt. Vorzugsweise weist der Handgriff auf einer dem Verbindungselement abgewandten Seite eine Skala auf, die unterschiedliche Schiebestellungen des Anschlagelements anzeigt. Vorzugsweise schlägt das Anschlagelement in zumindest eine Schiebestellung bei Verschwenken des Handgriffs an einer Außenfläche des (Milchbehälter-)Verbindungsabschnitts des Verbindungselements an. Es sind bevorzugt zumindest zwei, besonders bevorzugt zumindest drei, noch mehr bevorzugt zumindest vier Schiebestellungen vorgesehen. Vorzugsweise ist ein kontinuierliches Verschieben und Einstellen des Anschlagelements und damit eine kontinuierliche Verstellung des maximalen Unterdrucks vorgesehen. Das verschiebbare Anschlagelement erlaubt es, den zu erzeugenden Unterdruck einzustellen (bzw. zu begrenzen).

Im Folgenden wird die Erfindung anhand von in den Figuren dargestellten, besonders bevorzugten Ausführungsformen näher erläutert, auf die die Erfindung jedoch nicht beschränkt sein soll.
Fig. 1 zeigt schematisch eine bevorzugte Ausführungsform eines erfindungsgemäßen Milchpumpensystems in einer Explosionsdarstellung.
Fig. 2 zeigt schematisch dieselbe Ausführungsform des Milchpumpensystems wie Fig. 1 von der Seite.
Fig. 3a zeigt schematisch dieselbe Ausführungsform des Milchpumpensystems wie Fig. 1 in einer Schnittdarstellung.
Fig. 3b zeigt das Detail A der Fig. 3a.
Fig. 4a zeigt schematisch dieselbe Ausführungsform des Milchpumpensystems wie Fig. 1 mit einem Handgriff in einer ersten Drehstellung in einer Draufsicht.
Fig. 4b zeigt schematisch dieselbe Ausführungsform des Milchpumpensystems wie Fig. 1 mit dem Handgriff in einer zweiten Drehstellung in einer Draufsicht.
Fig. 4c zeigt schematisch dieselbe Ausführungsform des Milchpumpensystems wie Fig. 1 mit dem Handgriff in einer dritten Drehstellung in einer Draufsicht.
Fig. 5a und 5b zeigen schematisch eine zweite bevorzugte Ausführungsform eines Halteelements und eines Führungselements.
Fig. 6a und 6b zeigen schematisch eine dritte bevorzugte Ausführungsform des Halteelements und des Führungselements.
Fig. 7a zeigt schematisch eine bevorzugte Ausführungsform eines Verbindungselements in einer Schnittansicht.
Fig. 7b zeigt schematisch dieselbe Ausführungsform des Verbindungselements wie Fig. 7a in einer perspektivischen Ansicht.
Fig. 8a zeigt schematisch eine erste bevorzugte Ausführungsform eines Pumpelements in einer perspektivischen Ansicht.
Fig. 8b zeigt schematisch dieselbe Ausführungsform des Pumpelements wie Fig. 8a in einem Schnitt.
Fig. 9a zeigt schematisch eine weitere bevorzugte Ausführungsform eines des Milchpumpensystems mit einem Anschlagelement in einer ersten Schiebestellung in einer Ruhestellung in einer Seitenansicht.
Fig. 9b zeigt schematisch das gleiche Milchpumpensystem wie Fig. 9a mit dem Anschlagelement in der ersten Schiebestellung in einer Pumpstellung in einer Seitenansicht.
Fig. 9c zeigt schematisch das gleiche Milchpumpensystem wie Fig. 9a mit dem Anschlagelement in der ersten Schiebestellung in der Ruhestellung in einer perspektivischen Ansicht
Fig. 10a zeigt schematisch das gleiche Milchpumpensystem wie Fig. 9a mit dem Anschlagelement in einer zweiten Schiebestellung in einer Ruhestellung in einer Seitenansicht.
Fig. 10b zeigt schematisch das gleiche Milchpumpensystem wie Fig. 9a mit dem Anschlagelement in der zweiten Schiebestellung in einer Pumpstellung in einer Seitenansicht.
Fig. 10c zeigt schematisch das gleiche Milchpumpensystem wie Fig. 9a mit dem Anschlagelement in der zweiten Pumpstellung in einer Ruhestellung in einer perspektivischen Ansicht.
Fig. 11a zeigt schematisch eine zweite bevorzugte Ausführungsform eines Pumpelements in einer perspektivischen Ansicht.
Fig. 11b zeigt schematisch dieselbe Ausführungsform des Pumpelements wie Fig. 11a in einem Schnitt.
Fig. 12a zeigt schematisch das Milchpumpensystem der Ausführungsform der Fig. 1 in einer alternativen Verwendungsmöglichkeit in einer Seitenansicht.
Fig. 12b zeigt schematisch das Milchpumpensystem in der alternativen Verwendungsmöglichkeit der Fig. 12a von unten.
Fig. 12c zeigt schematisch das Milchpumpensystem in der alternativen Verwendungsmöglichkeit der Fig. 12a in einer Schnittdarstellung entlang der Schnittlinie A-A in Fig. 12b.
Fig. 12d zeigt das Detail B der Fig. 12c.

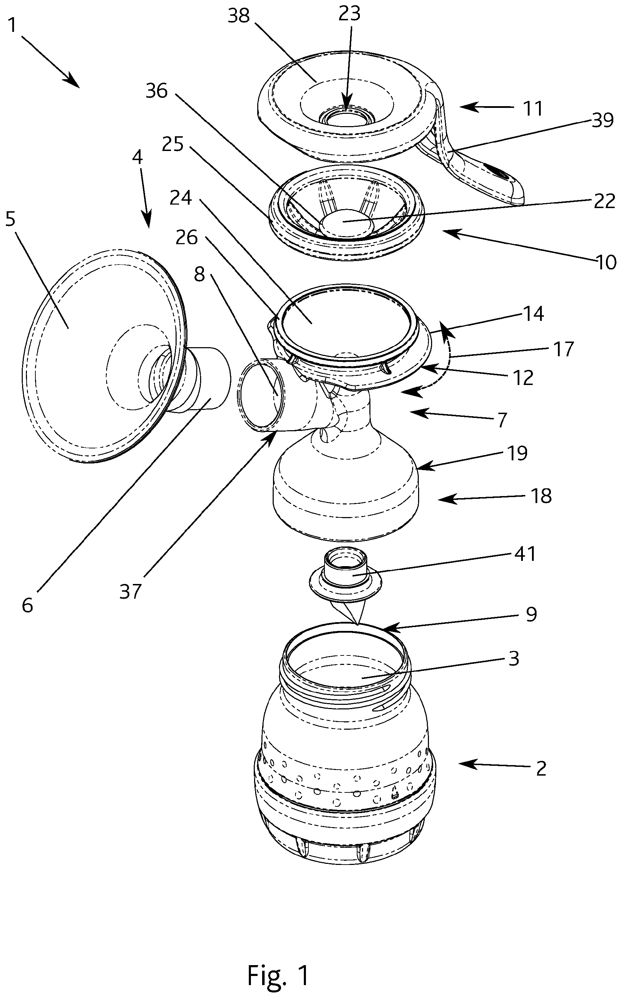

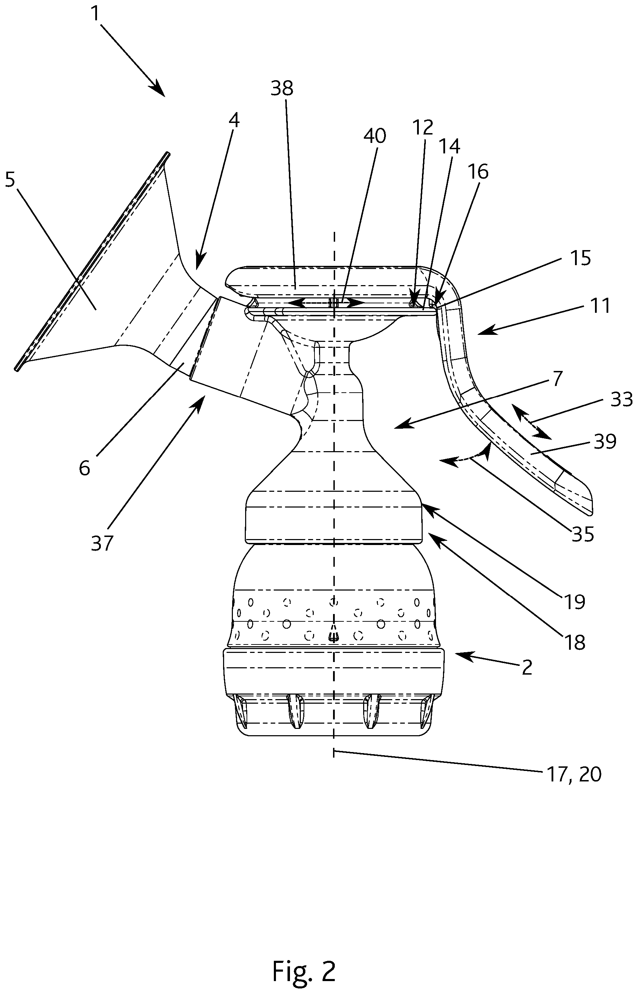

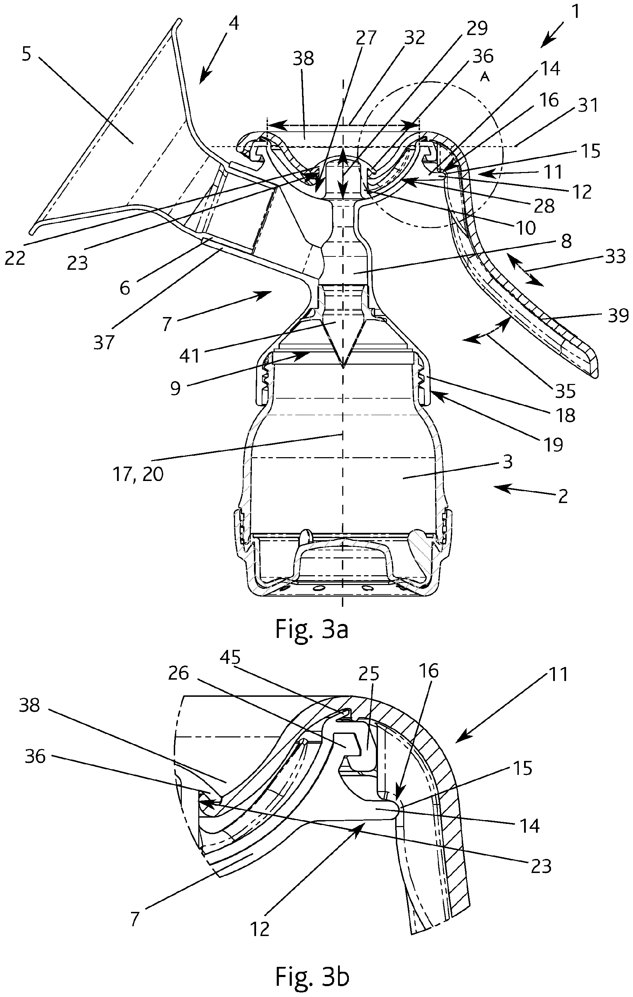

Fig. 1 zeigt schematisch eine bevorzugte Ausführungsform eines erfindungsgemäßen Milchpumpensystem 1 in einer Explosionsdarstellung. Fig. 2 zeigt dasselbe Milchpumpensystem 1 zusammengebaut von der Seite. Fig. 3a zeigt dasselbe Milchpumpensystem 1 in einer Schnittdarstellung. Fig. 3b zeigt das Detail A der Fig. 3a.

Das Milchpumpensystem 1 ist verbunden mit einem (vorzugsweise externen) Milchbehälter 2 mit einem Hohlraum 3 zur Aufnahme von abgepumpter Milch und einer (vorzugsweise externen) Brusthaube 4, die einen Brusttrichter 5 und einen Nippeltunnel 6 aufweist, dargestellt. Der Milchbehälter 2 und die Brusthaube 4 sind vorzugsweise jeweils lösbar mit dem Milchpumpensystem 1 verbunden. Das Milchpumpensystem 1 kann auch den Milchbehälter 2 und die Brusthaube 4 aufweisen. Das Milchpumpensystem 1 ist dann vorzugsweise eine Milchpumpe. Es kann auch ein Einsatz für den Brusttrichter 5 vorgesehen sein (nicht dargestellt), der insbesondere dazu dienen kann, den Durchmesser des Brusttrichters 5 zur Aufnahme verschieden großer Brustwarzen zu variieren. Das Milchpumpensystem 1 weist weiter ein Verbindungselement 7 zur Verbindung des Milchbehälters 2 mit der Brusthaube 4, wobei das Verbindungselement 7 einen Kanal 8 zur (insbesondere fluidischen) Verbindung einer Öffnung 9 zum Hohlraum 3 des Milchbehälters 2 mit dem Nippeltunnel 6 der Brusthaube 4 aufweist, ein Pumpelement 10, das dazu eingerichtet ist, einen Unterdruck im Kanal 8 des Verbindungselements 7 zu erzeugen, und einen Handgriff 11, der dazu eingerichtet ist, das Pumpelement 10 zu betätigen, auf. Das Milchpumpensystem 1 weist im Kanal 8 ein Rückschlagventil 41 auf. Das Verbindungselement 7 weist ein Führungselement 12 in Form einer länglich verlaufenden Auskragung 14 auf und der Handgriff 11 weist ein Halteelement 15 in Form einer Einbuchtung (und eines daran anschließenden Vorsprungs) auf, wobei das Halteelement 15 und das Führungselement 12 miteinander in Eingriff stehen (also insbesondere die längliche verlaufende Auskragung 14 in die Einbuchtung eingreift), sodass der Handgriff 11 um die Eingriffsstelle 16 zum Betätigen des Pumpelements 10 schwenkbar ist. Beim Schwenken stützt sich somit der Handgriff 11 mittels des Halteelements 15 direkt am Führungselement 12 des Verbindungselements 7 ab. Damit liegt eine Schwenkachse, um die beim Pumpen der Handgriff 11 geschwenkt wird, nahe am Verbindungselement 7 bzw. dessen Zentrum, wodurch der notwendige Hub bzw. der beim Pumpen zu umfassende Abstand gering sind. Die Schwenkachse verläuft durch die Eingriffsstelle 16.

In Fig. 2 und 3 ist der Handgriff 11 in einer Ruhestellung zu sehen. Der Handgriff 11 weist einen Halteabschnitt 38 zur Verbindung mit dem Pumpelement 10 und einen Hebelarm 39 (zur Betätigung durch einen Benutzer des Milchpumpensystems 1) auf. Die Schwenkachse verläuft insbesondere im Wesentlichen senkrecht zur Längserstreckungsrichtung 33 des Hebelarms 39. Der Halteabschnitt 38 und der Hebelarm 39 sind winkelig miteinander verbunden. Zum Betätigen des Pumpelements 10 wird der Hebelarm 39 in Richtung des Verbindungselements 7 verschwenkt.

Insbesondere sind zwei entsprechende Halteelemente 15 vorgesehen (in Fig. 1 bis 3 nicht zu sehen), die parallel zueinander sind und gleichermaßen ausgebildet sind. Alternativ können die zwei Halteelemente 15 auch gewinkelt zueinander stehen. Diese greifen in Umfangsrichtung des Verbindungselements 7 voneinander beabstandet in das Führungselement 12 ein, wobei die Schwenkachse durch beide entsprechende Eingriffsstellen 16 verläuft. Die folgenden Angaben für ein Halteelement 15 beziehen sich somit auf beide Halteelemente 15.

Beim Zusammenbauen des Milchpumpensystems 1 wird das Halteelement 15 (bzw. im Fall mehrerer Halteelemente 15: werden die Halteelemente 15) an der gewünschten Stelle mit dem Führungselement 12 in Eingriff gebracht. Das Halteelement 15 ist entlang des Führungselements 12 verschiebbar. Im zusammengebauten Zustand kann der Handgriff 11 gedreht werden, in dem das Halteelement 15 bzw. die Halteelemente 15 entlang des Führungselements 12 verschoben werden. Das Führungselement 12 erstreckt sich im Wesentlichen entlang eines Kreisbogens, wodurch sich der (Ruhe-)Schwenkwinkel des Handgriffs 11 beim Verdrehen nicht verändert. (Alternativ ist bzw. auch ein Verlauf des Führungselements 12 entlang eines Polygonzugs denkbar. Dadurch könnte der Handgriff 11 in definierte Drehwinkelstellungen gebracht werden.) In dieser Ausführungsform verläuft das Führungselement 12 in Umfangsrichtung 40 des Verbindungselements 7 ungefähr über den größtmöglichen Winkelbereich beidseits des Brusthauben-Verbindungsabschnitts 37 des Verbindungselements 7, im Konkreten über einen Winkelbereich von ca. 280°. Es ist somit ein Verdrehen des Handgriffs 11 über einen großen Winkelbereich um die Drehachse 17 möglich. Das Verbindungselement 7 weist einen Verbindungsabschnitt 18 zur Befestigung des Milchbehälters 2 auf, wobei der Verbindungsabschnitt 18 im Wesentlichen eine rotationssymmetrische Mantelfläche 19 aufweist. Die Drehachse 17 des Handgriffs 11 fällt mit der Rotationssymmetrieachse 20 der Mantelfläche 19 zusammen. Der maximale Pumphub (d.h. der maximale Schwenkwinkel des Handgriffs 11) wird insbesondere vorgegeben und begrenzt durch ein Anstoßen des Handgriffs 11 am Verbindungselement 7, insbesondere an der rotationssymmetrischen Mantelfläche 19 des Verbindungselements 7. Da die Drehachse 17 mit der Rotationssymmetrieachse 20 gleich ist, ist somit unabhängig von der Drehstellung des Handgriffs 11 immer der gleiche Maximalhub und damit das gleiche Maximalvakuum vorgegeben.

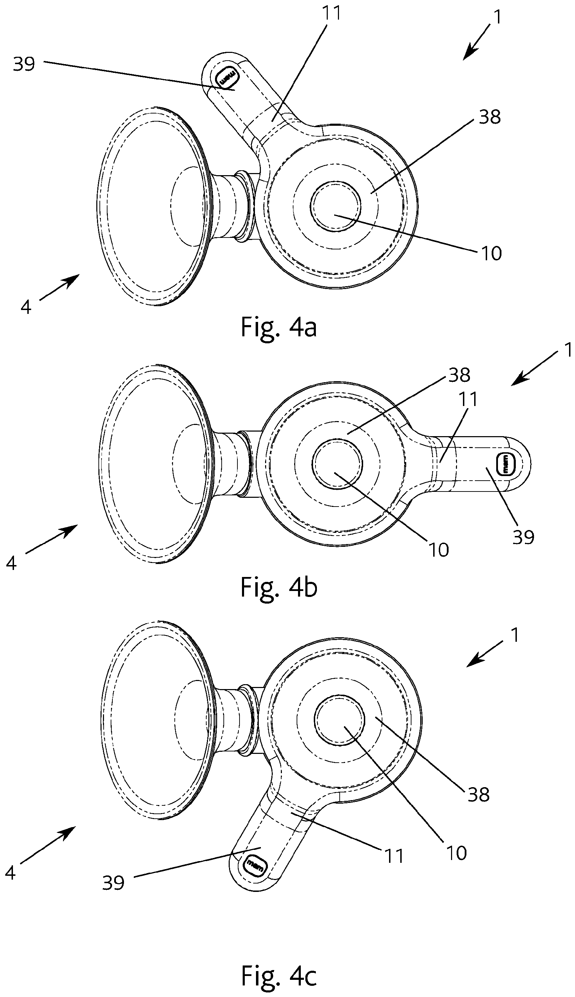

Die Fig. 4a bis 4c zeigen dasselbe Milchpumpensystem 1 mit dem Handgriff 11 in unterschiedlichen Drehstellungen. In Fig. 4b ist der Handgriff 11 in einer mittleren Drehstellung, in der der Hebelarm 39 der Brusthaube 4 gegenüberliegt.

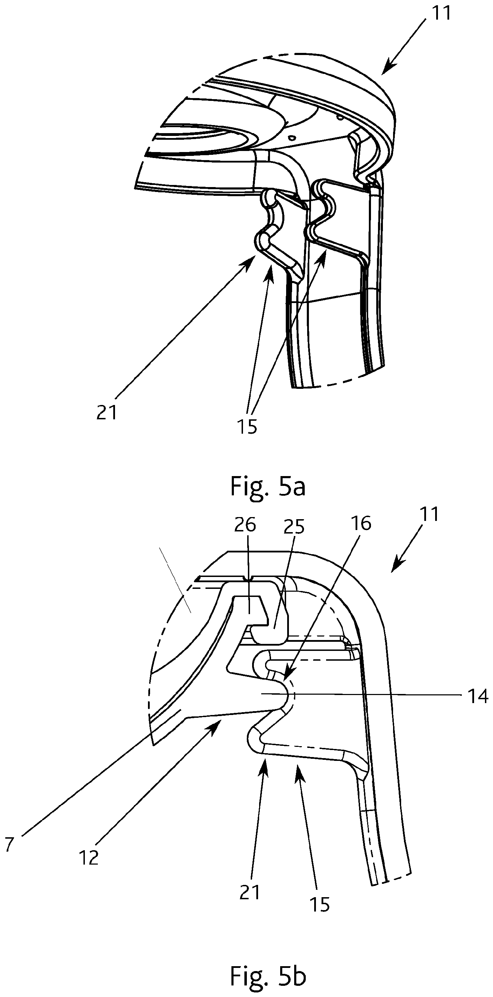

Eine alternative Ausführungsform des Halteelements 15 (bzw. der zwei Halteelemente 15) und des Führungselements 12 (die auch bei der Ausführungsform der Fig. 1 verwendet werden könnten) sind in den Fig. 5a und 5b zu sehen. In dieser Ausführungsform ist das Halteelement 15 als Haltevorsprung ausgeführt und weist ein gabelförmiges Endstück 21 auf, mit dem das Führungselement 12 in Eingriff steht. Die beiden Arme des gabelförmigen Endstücks 21 umgreifen dabei die länglich verlaufende Auskragung 14.

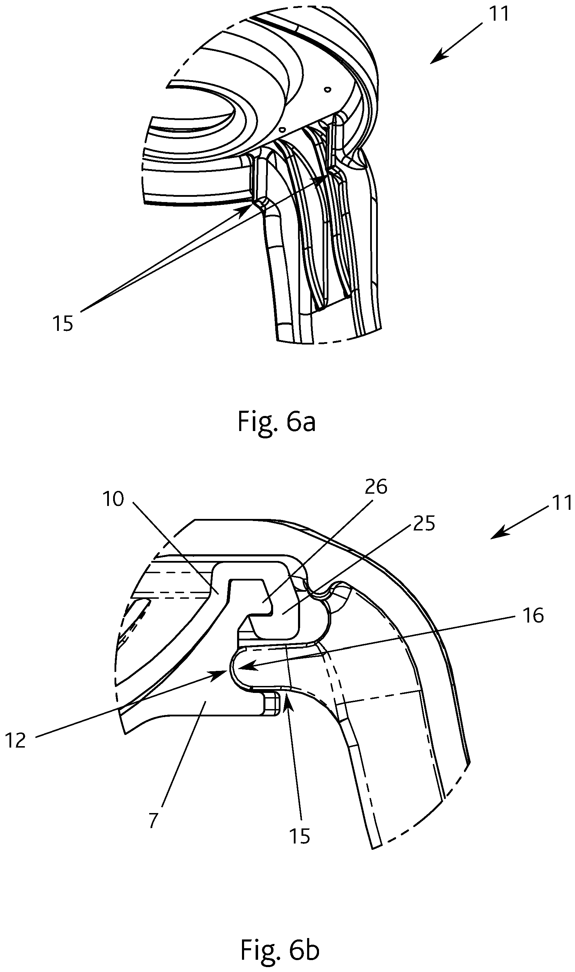

Eine weitere alternative Ausführung des Halteelements 15 (bzw. der zwei Halteelemente 15) und des Führungselements 12 sind in Fig. 6a und 6b zu sehen. Diese Ausführung kann auch als Alternative bei dem Milchpumpensystem 1 der Fig. 1 verwendet werden. In dieser Ausführung ist das Halteelement 15 in Form eines Haltevorsprungs als einzelner Arm ausgebildet. Das Führungselement 12 weist eine Nut auf, in die das Halteelement 15 eingreift.

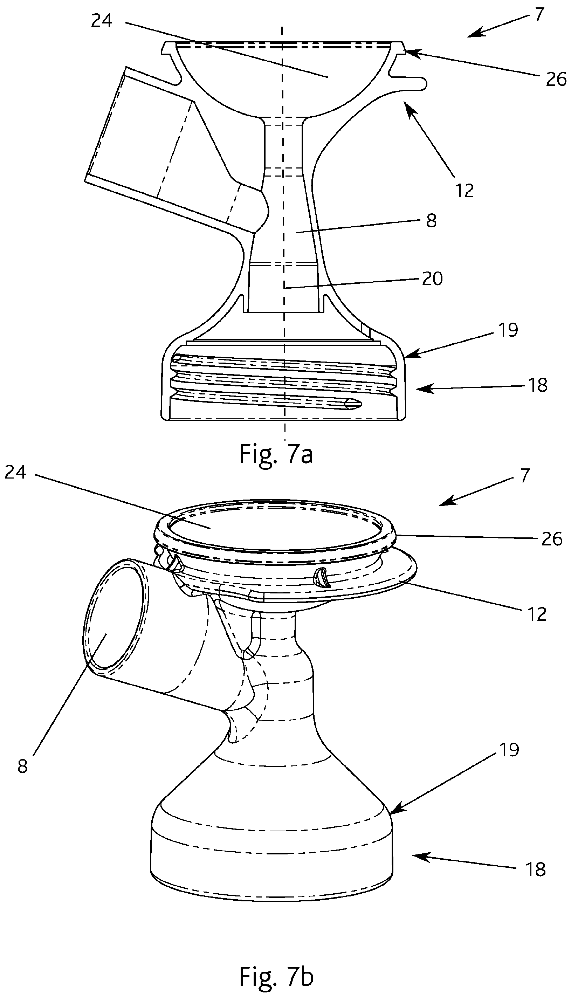

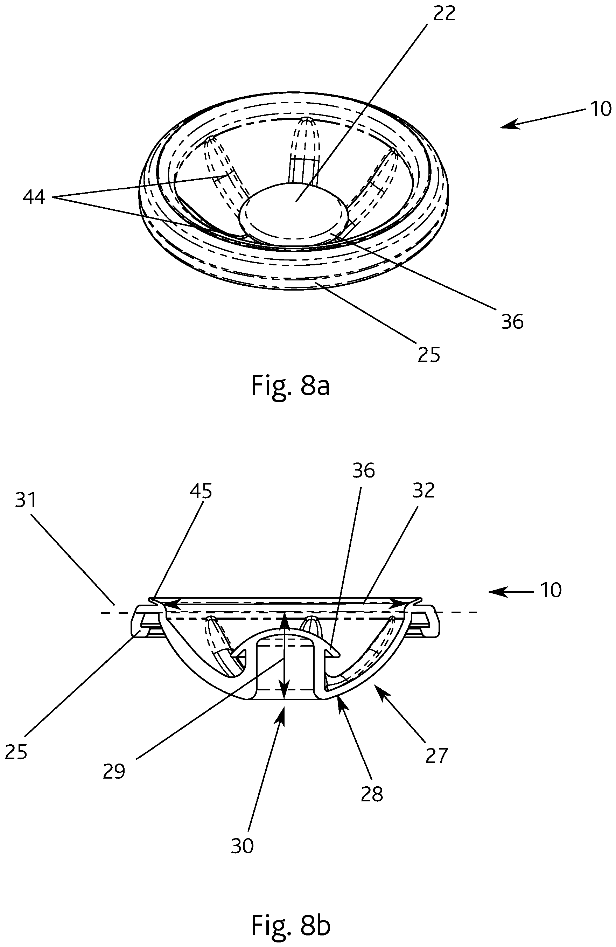

Das Verbindungselement 7 des Milchpumpensystems 1 der Fig. 1 ist in den Figuren 7a und 7b zur besseren Veranschaulichung alleine dargestellt. Das Pumpelement 10 des Milchpumpensystems 1 der Fig 1. ist in den Figuren 8a und 8b zur besseren Veranschaulichung alleine dargestellt.

Das Verbindungselement 7 bildet einen im Wesentlichen schalenförmigen Aufnahmeraum 24 für das Pumpelement 10 aus, der mit dem Kanal 8 verbunden ist, wobei das Pumpelement 10 den Aufnahmeraum 24 im Wesentlichen abdeckt. Dabei weist das Pumpelement 10 einen umlaufenden Schnapprand 25 auf, der mit einem den Aufnahmeraum 24 begrenzenden umlaufenden Befestigungsrand 26 des Verbindungselements 7 in Schnappverbindung steht. Damit wird einerseits das Pumpelement 10 beim Pumpen am Verbindungselement 7 gehalten. Andererseits wird der zu evakuierende Raum zwischen Verbindungselement 7 und Pumpelement 10 auch zur Umgebung hin abgedichtet. Das Pumpelement 10 liegt in einer Ruhestellung des Handgriffs 11 am Verbindungselement 7 im Bereich des Aufnahmeraums 24 im Wesentlichen an. Damit wird die Größe des zu evakuierenden Raums möglichst klein gehalten, wodurch der erforderliche Hub verringert wird.

Das Pumpelement 10 weist einen Haltezapfen 22 und der Handgriff 11 eine Halteöffnung 23 auft, wobei der Haltezapfen 22 durch die Halteöffnung 23 geführt ist und der Haltezapfen 22 eine Hinterschneidung 36 aufweist, um den Handgriff 11 zu halten. Ein Verschwenken des Handgriffs 11 um die Eingriffsstelle 16 bewirkt somit ein Betätigen des Pumpelements 10. Beim Verdrehen des Handgriffs 11 rotiert die Halteöffnung 23 um den Haltezapfen 22. Das Pumpelement 10 weist einen schalenförmigen Abschnitt 27 auf. Durch eine Verformung des schalenförmigen Abschnitts 27 wird der Unterdruck im Kanal 8 des Verbindungselements 7 erzeugt. Beim Verschwenken des Handgriffs 11 wird der Haltezapfen 22 von der Halteöffnung 23 nach oben (weg von dem Verbindungselement 7 bzw. dem Milchbehälter 2) gezogen. Der Haltezapfen 22 bewirkt einen Zug an dem schalenförmigen Abschnitt 27 nach oben, wodurch der Unterdruck erzeugt wird. Das Pumpelement 10 (insbesondere der Haltezapfen 22 und eine Membran, die den schalenförmigen Abschnitt 27 bildet) ist insbesondere einstückig ausgeführt. Das Pumpelement 10 kann auch ein Griffelement aufweisen (in dieser Ausführungsform nicht gezeigt), mit dem die Montage vereinfacht wird.

Eine dem Verbindungselement 7 zugewandte Außenfläche 28 des Pumpelements 10 im schalenförmigen Abschnitt 27 ist im Wesentlichen rotationssymmetrisch. Vorzugsweise ist auch der Aufnahmeraum 24 des Verbindungselements 7 rotationssymmetrisch ausgeführt. Der schalenförmige Abschnitt 27 des Pumpelements 10 weist eine Tiefe 29, gemessen von einer Spitze 30 des schalenförmigen Abschnitts 27 zu einer Ebene 31, in der ein Rand des schalenförmigen Abschnitts 27 liegt, und eine Breite 32, die der größten Erstreckung in der Ebene 31, in der der Rand des schalenförmigen Abschnitts 27 liegt, entspricht, auf. Die Breite 32 ist mehr als doppelt so groß wie die Tiefe 29. Damit wird die erforderliche Hubhöhe verringert.

Das Pumpelement 10 weist eine Verrippung 44 in Form von Rippen zur Verstärkung des schalenförmigen Abschnitts 27 auf. Diese ermöglichen es, einen stärkeren Unterdruck bei kurzem Hub zu erzielen und eine Rückstellung des Handgriffs 11 auch ohne Vakuum zu ermöglichen.

Zum Zusammenbau der Brustpumpe wird der Haltezapfen 22 des Pumpelements 10 mit der Hinterschneidung 36 durch die Halteöffnung 23 des Handgriffs 11 gezogen/gedrückt, um das Pumpelement 10 am Handgriff 11 zu befestigen. Dies wird vereinfacht, indem eine dem Milchbehälter 2 bzw. dem Verbindungselement 7 abgewandte Oberseite des Haltezapfens 22 (insbesondere anschließend an die Hinterschneidung 36) geschlossen ist. Durch die geschlossene Oberseite wird das Verbindungselement 7 auch im Bereich der Hinterschneidung 36 versteift. Der Handgriff 11 wird mit dem Pumpelement 10 am Verbindungselement 7 aufgesetzt. Dabei wird der Schnapprand 25 über den Befestigungsrand 26 gezogen, und das Halteelement 15 und das Führungselement 12 miteinander in Eingriff gebracht.

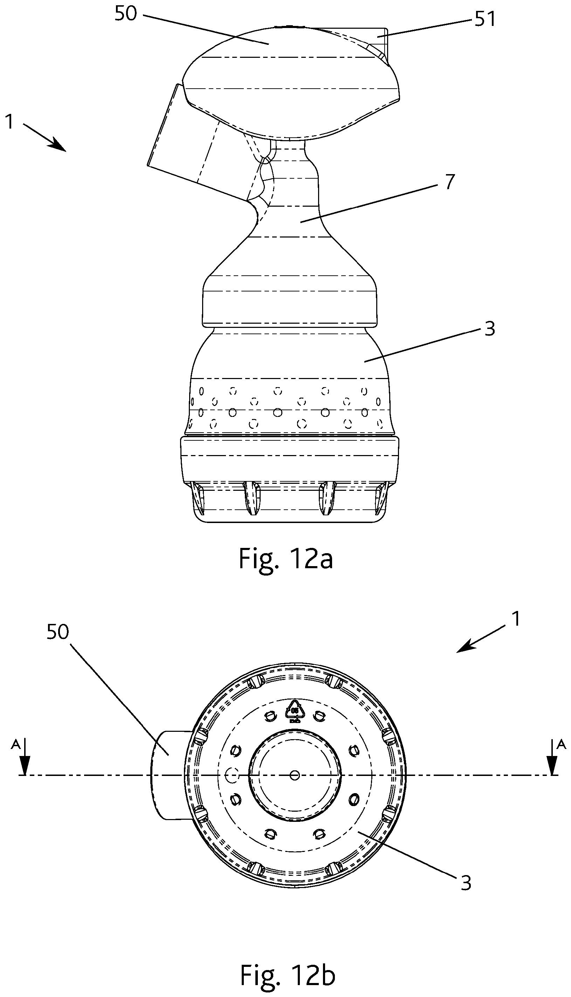

Das Pumpelement 10 weist weiters eine insbesondere vollumfänglich verlaufende Dichtlippe 45 auf. Die Dichtlippe 45 erstreckt sich insbesondere von einem umfänglichen Flansch 46 des Pumpelements 10, der an jenen Abschnitt des Pumpelements 10 anschließt bzw. jenen Abschnitt des Pumpelements 10 begrenzt, der den Aufnahmeraum 24 abdeckt oder im Aufnahmeraum 24 liegt. An den umlaufenden Flansch 46 wiederum schließt der Schnapprand 25 an. Die Dichtlippe 45 dient insbesondere dem Anschluss einer elektrischen Pumpe, wie im Zusammenhang mit den Fig. 12a bis 12d näher beschrieben wird.

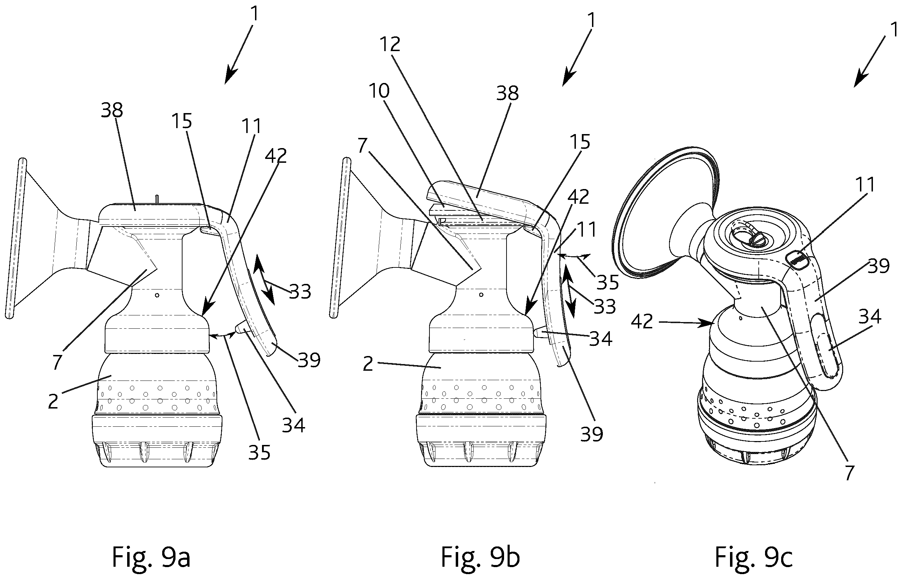

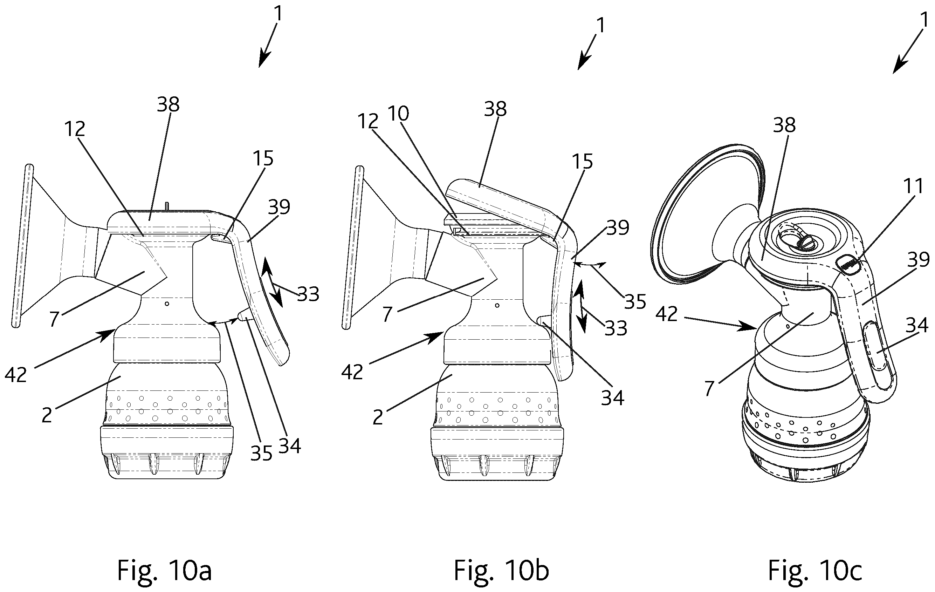

Die Fig. 9a bis 10c zeigen eine weitere bevorzugte Ausführungsform des Milchpumpensystems. Diese ist im Wesentlichen gleich ausgebildet wie das in Fig. 1 dargestellte, jedoch weist bei dieser Ausführungsform zusätzlich der Handgriff 11 zur Vakuumbegrenzung ein in Längserstreckungsrichtung 33 (insbesondere lokal betrachtet) des Handgriffs 11 (bzw. des Hebelarms 39 des Handgriffs 11) verschiebbares Anschlagelement 34 auf. Der Handgriff 11 ist in den Fig. 9a, 9c, 10a und 10c in einer Ruhestellung und in den Fig. 9b und 10b in der maximal verschwenkten Stellung (in der also das Pumpelement 10 maximal betätigt ist) dargestellt. In dieser Ausführungsform ist das Anschlagelement 34 im Wesentlichen zwischen einer ersten und einer zweiten Schiebestellung verschiebbar. Die Fig. 9a bis 9c zeigen das Anschlagelement 34 in der ersten Schiebestellung und die Fig. 10a bis 10c das Anschlagelement 34 in der zweiten Schiebestellung. Das Verbindungselement 7 weist einen Abschnitt 42 mit zunehmendem Querschnitt auf. Der Außenquerschnitt (bzw. Durchmesser) nimmt dabei im Abschnitt 42 nach unten hin zu. In der ersten Schiebestellung schlägt das Anschlagelement 34 beim Verschwenken des Handgriffs 11 an einer ersten (unteren) Stelle des Verbindungselements 7 an, sodass das Anschlagelement den maximalen Schwenkwinkel 35 des Handgriffs 11 und damit den mit jeder Pumpbewegung erzeugten Unterdruck (maximal) auf einen ersten Schwenkwinkel begrenzt. In der zweiten Schiebestellung erfolgt beim Verschwenken des Handgriffs 11 ein Anschlagen des Anschlagselements 34 an einer zweiten (oberen) Stelle des Abschnitts 42 des Verbindungselements 7, an dem der Durchmesser des Verbindungselements 7 geringer ist. Somit wird in der zweiten Schiebestellung der maximale Schwenkwinkel auf einen zweiten Schwenkwinkel begrenzt, der größer als der erste Schwenkwinkel ist. Alternativ könnte in der zweiten Schiebestellung das Anschlagelement 34 nicht am Verbindungselement 7 anschlagen, sodass der maximale Schwenkwinkel nur durch ein Anschlagen des Hebelarms 39 am Verbindungselement 7 begrenzt wäre. Der maximale Schwenkwinkel des Handgriffs 11 und damit der erzeugte Unterdruck sind somit in der zweiten Schiebestellung größer als in der ersten Schiebestellung.

In einer modifizierten (nicht dargestellten) Ausführungsform der Fig. 9a bis 10c können für das Anschlagelement 34 drei oder mehr Schiebestellungen vorgesehen sein. Wie auch in Fig. 9a zu sehen, weist das Verbindungselement 7 einen Abschnitt 42 mit zunehmendem Querschnitt auf. In der modifizierten Ausführungsform ist das Anschlagelement 34 in (insbesondere lokal betrachteter) Längserstreckungsrichtung 33 des Handgriffs 11 zumindest teilweise entlang des Abschnitts 42 mit zunehmendem Außenquerschnitt verschiebbar, sodass das Anschlagelement 34 beim Verschwenken des Handgriffs 11 in der ersten Schiebestellung an einer ersten (unteren) Stelle des Abschnitts 42 mit zunehmendem Außenquerschnitt anschlägt und in der zweiten Schiebestellung an einer zweiten (weiter oberen) Stelle des Abschnitts 42 mit zunehmendem Querschnitt anschlägt. Der Außenquerschnitt nimmt dabei im Abschnitt 42 nach unten hin zu. Das Anschlagelement 34 begrenzt somit in der ersten Schiebestellung den Schwenkwinkel des Handgriffs 11 auf einen ersten Winkel und in der zweiten Schiebestellung auf einen zweiten Winkel, der größer als der erste Winkel ist. In der dritten Schiebestellung schlägt das Anschlagelement 34 nicht am Verbindungselement 7 an, sondern der maximale Schwenkwinkel wird durch das Anschlagen des Handgriffs 11 am Verbindungselement 7 begrenzt. Es sind somit drei (oder mehr) unterschiedliche Schwenkwinkel und damit erzeugbare Unterdrücke einstellbar. Der Handgriff 11 (insbesondere der Hebelarm 39) kann außerdem auf einer dem Verbindungselement 7 abgewandten Seite eine Skala aufweisen, die unterschiedliche Schiebestellungen des Anschlagelements anzeigt.

Es ist auch eine kontinuierliche Einstellung der Schiebestellung des Anschlagelements 34 möglich.

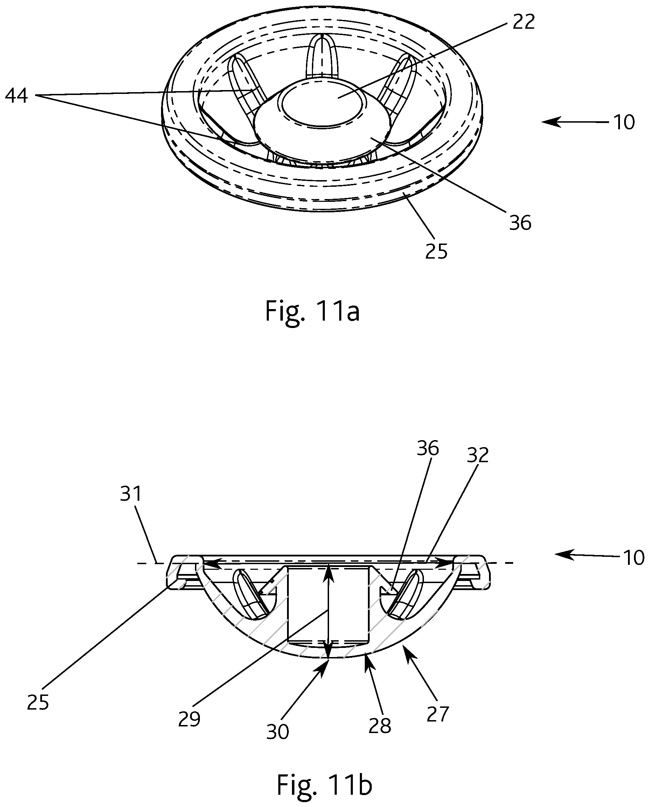

Die Fig. 11a zeigt schematisch eine weitere bevorzugte Ausführungsform des Pumpelements 10 in einer perspektivischen Ansicht; Fig. 11b zeigt schematisch dieselbe Ausführungsform in einem Schnitt. Diese kann beispielsweise beim Milchpumpensystem 1 anstelle der in den Fig. 8a und 8b dargestellten Ausführungsform des Pumpelements 10 verwendet werden. Diese Ausführungsform des Pumpelements 10 ist im Wesentlichen gleich aufgebaut wie jene der Fig. 8a und 8b, jedoch ist im Unterschied dazu nicht die dem Milchbehälter 2 bzw. dem Verbindungselement 7 abgewandte Oberseite des Haltezapfens 22, sondern eine dem Milchbehälter 2 bzw. dem Verbindungselement 7 zugewandte Unterseite des Haltezapfens 22 geschlossen. Dadurch kann der zu evakuierende Raum verkleinert werden. Bei dieser Ausführungsform ist auch keine Dichtlippe 45 dargestellt; diese kann jedoch auch hier vorgesehen sein.

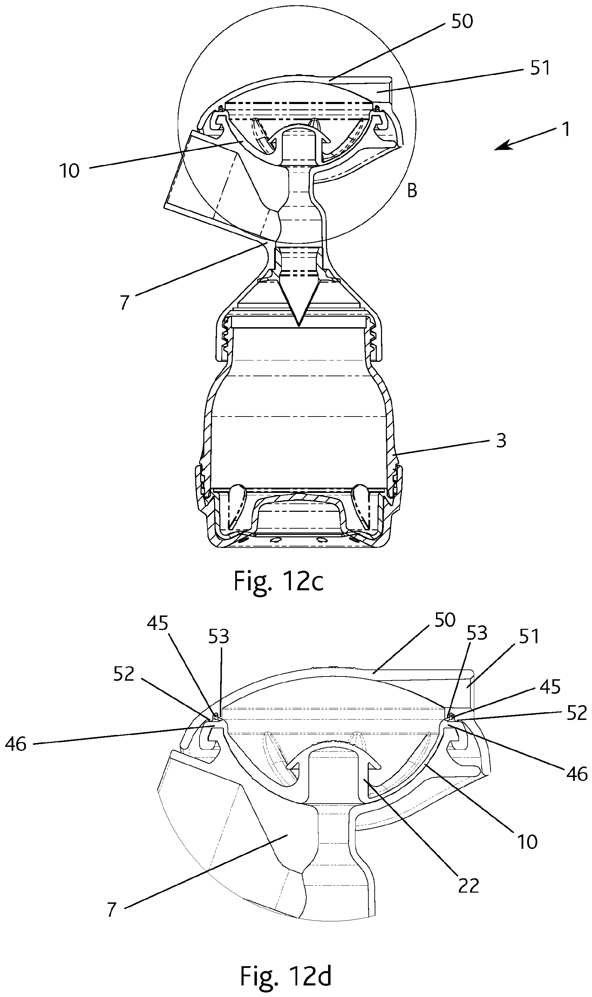

Fig. 12a zeigt schematisch eine alternative Verwendungsmöglichkeit des Milchpumpensystems 1 der Ausführungsform der Fig. 1 in einer Seitenansicht; Fig. 12b in einer Ansicht von unten, Fig. 12c in einer Schnittdarstellung entlang der Schnittlinie A-A in Fig. 12b und Fig. 12d das Detail B der Fig. 12c. In der alternativen Verwendungsmöglichkeit kann das Milchpumpensystem 1, insbesondere der Verbindungsabschnitt 7 und das Pumpelement 10, auch für eine elektrische Milchpumpe verwendet werden.

In dieser Verwendungsmöglichkeit des Milchpumpensystems 1 wurde der Handgriff 11 entfernt. (Die Brusthaube 4 ist in den Fig. 12a bis 12d ebenfalls entfernt). Statt des Handgriffs 11 ist eine Vakuumhaube 50 zur Verbindung mit einer Pump-Motoreinheit (nicht dargestellt) auf dem Pumpelement 10 aufgesetzt. Die Vakuumhaube 50 bildet über dem Pumpelement 10 einen abgeschlossenen Raum, wobei ein Schlauchanschluss 51 für den Anschluss der Pump-Motoreinheit vorgesehen ist. Durch Erzeugen eines Unterdrucks/Vakuums im abgeschlossenen Raum durch die Pump-Motoreinheit kann somit das Pumpelement 10 betätigt und Milch gepumpt werden.

Die Vakuumhaube 50 weist vorzugsweise einen ersten umlaufenden Vorsprung 52 und einen zweiten umlaufenden Vorsprung 53 auf. Es ist bevorzugt, wenn der erste umlaufende Vorsprung 52 (insbesondere mit einer umlaufenden Spitze des umlaufenden Vorsprungs 52) am umlaufenden Flansch 46 des Pumpelements 10 anliegt und/oder eine Spitze der Dichtlippe 45 am ersten umlaufenden Vorsprung 52, insbesondere an einer Seitenwand des ersten umlaufenden Vorsprungs 52, anstößt. Es ist bevorzugt, wenn der zweite umlaufende Vorsprung 53 (insbesondere eine umlaufende Spitze des zweiten umlaufenden Vorsprungs 53) auf der Dichtlippe 45 aufliegt. Die Dichtlippe 45 dient, insbesondere zusammen mit dem ersten umlaufenden Vorsprung 52 und/oder dem zweiten umlaufenden Vorsprung 53, dazu, den abgeschlossenen Raum (bis auf den Schlauchanschluss 51) gegenüber der Umgebung abzudichten. Unter steigendem Vakuumeinfluss saugt sich die Vakuumhaube 50 somit immer stärker gegen die Dichtlippe 45 bzw. das Pumpelement 10.

Zum Umbau vom Aufbau der Fig. 1 in diese alternative Verwendungsmöglichkeit als elektrische Milchpumpe wird zuerst der Handgriff 11 entfernt (bzw. beim erstmaligen Zusammenbau das Pumpelement 10 auf das Verbindungselement 7 geschnappt). Anschließend wird die Vakuumhaube 50 auf dem Verbindungselement 7 und dem Pumpelement 10 aufgesetzt (bspw. verschnappt oder mittels Bajonettverbindung oder Gewindeverbindung befestigt). Die Dichtlippe 45 wird dabei dichtend gegen die Vakuumhaube 50 gedrückt, sodass sich diese unter steigendem Vakuumeinfluss wie oben beschrieben immer stärker am Pumpelement 7 ansaugt. Das Maximalvakuum kann durch die Pump-Motoreinheit, und/oder durch mechanische Anschläge (Rippen, Ringstege, etc.) innerhalb der Vakuumhaube 50 (von der Innenkuppel nach unten ragend, hier nicht dargestellt) gesteuert werden.

## Patentansprüche

1. Milchpumpensystem (1) aufweisend
- ein Verbindungselement (7) zur Verbindung eines Milchbehälters (2) mit einer Brusthaube (4), wobei das Verbindungselement (7) einen Kanal (8) zur Verbindung eines Hohlraums (3) des Milchbehälters (2) mit einem Nippeltunnel (6) der Brusthaube (4) aufweist;
- ein Pumpelement (10), das dazu eingerichtet ist, einen Unterdruck im Kanal (8) des Verbindungselements (7) zu erzeugen;
- einen Handgriff (11), der dazu eingerichtet ist, das Pumpelement (10) zu betätigen;
**dadurch gekennzeichnet, dass** das Verbindungselement (7) ein Führungselement (12) in Form einer Nut oder länglich verlaufenden Auskragung (14) aufweist und der Handgriff (11) ein Halteelement (15) aufweist, wobei das Halteelement (15) und das Führungselement (12) miteinander in Eingriff stehen, sodass der Handgriff (11) um die Eingriffsstelle (16) zum Betätigen des Pumpelements (10) schwenkbar ist.

2. Milchpumpensystem (1) nach Anspruch 1, wobei das Halteelement (15) entlang des Führungselements (12) verschiebbar ist.

3. Milchpumpensystem (1) nach einem der vorhergehenden Ansprüche, wobei sich das Führungselement (12) im Wesentlichen entlang eines Kreisbogens oder eines Polygonzugs erstreckt.

4. Milchpumpensystem (1) nach einem der vorhergehenden Ansprüche, wobei das Führungselement (12) in Umfangsrichtung (40) des Verbindungselements (7) über einen Winkelbereich von zumindest 30°, bevorzugt zumindest 90°, besonders bevorzugt zumindest 180° noch mehr bevorzugt zumindest 250°, noch weiter bevorzugt zumindest 270°, verläuft.

5. Milchpumpensystem (1) nach einem der vorhergehenden Ansprüche, wobei der Handgriff (11) durch Verschieben des Halteelements (15) entlang des Führungselements (12) drehbar um eine Drehachse (17) ist, wobei vorzugsweise der maximale Winkel, um den der Handgriff (11) zum Betätigen des Pumpelements (12) schwenkbar ist, unabhängig von der Drehstellung des Handgriffs (11) um die Drehachse (17) ist.

6. Milchpumpensystem (1) nach Anspruch 5, wobei das Verbindungselement (7) einen Verbindungsabschnitt (18) zur Befestigung des Milchbehälters (2) aufweist, wobei der Verbindungsabschnitt (18) im Wesentlichen eine rotationssymmetrische Mantelfläche (19) aufweist, wobei die Drehachse (17) parallel zu einer Rotationssymmetrieachse (20) der Mantelfläche (19) ist, wobei insbesondere die Drehachse (17) und die Rotationssymmetrieachse (20) zusammenfallen.

7. Milchpumpensystem (1) nach einem der vorhergehenden Ansprüche, wobei das Halteelement (15) eine Einbuchtung aufweist, mit der das Führungselement (12) in Eingriff steht.

8. Milchpumpensystem (1) nach einem der vorhergehenden Ansprüche, wobei das Pumpelement (10) einen Haltezapfen (22) und der Handgriff (11) eine Halteöffnung (23) aufweist, wobei der Haltezapfen (22) durch die Halteöffnung (23) geführt ist und eine Hinterschneidung (36) aufweist, um den Handgriff (11) zu halten, sodass ein Verschwenken des Handgriffs (11) um die Eingriffsstelle (16) ein Betätigen des Pumpelements (10) bewirkt.

9. Milchpumpensystem (1) nach einem der vorhergehenden Ansprüche, wobei das Verbindungselement (7) einen Aufnahmeraum (24) für das Pumpelement (10) ausbildet, der mit dem Kanal (8) verbunden ist, wobei das Pumpelement (10) den Aufnahmeraum (24) im Wesentlichen abdeckt.

10. Milchpumpensystem (1) nach Anspruch 9, wobei das Pumpelement (10) einen umlaufenden Schnapprand (25) aufweist, der mit einem den Aufnahmeraum (24) begrenzenden umlaufenden Befestigungsrand (26) des Verbindungselements (7) in Schnappverbindung steht.

11. Milchpumpensystem (1) nach einem der Ansprüche 9 oder 10, wobei das Pumpelement (10) in einer Ruhestellung des Handgriffs (11) am Verbindungselement (7) im Bereich des Aufnahmeraums (24) im Wesentlichen anliegt.

12. Milchpumpensystem (1) nach einem der vorhergehenden Ansprüche, wobei das Pumpelement (10) einen schalenförmigen Abschnitt (27) aufweist, wobei durch eine Verformung des schalenförmigen Abschnitts (27) der Unterdruck im Kanal (8) des Verbindungselements (7) erzeugbar ist.

13. Milchpumpensystem (1) nach Anspruch 12, wobei eine dem Verbindungselement (7) zugewandte Außenfläche (28) des Pumpelements (10) im schalenförmigen Abschnitt (27) im Wesentlichen rotationssymmetrisch ist.

14. Milchpumpensystem (1) nach einem der Ansprüche 12 oder 13, wobei der schalenförmige Abschnitt (27) des Pumpelements (10) eine Tiefe (29) aufweist, gemessen von einer Spitze (30) des schalenförmigen Abschnitts (27) zu einer Ebene (31), in der ein Rand des schalenförmigen Abschnitts (27) liegt, und der schalenförmige Abschnitt (27) eine Breite (32) aufweist, die der größten Erstreckung in der Ebene (31), in der der Rand des schalenförmigen Abschnitts (27) liegt, entspricht, wobei die Breite (32) mindestens 1,5 mal groß wie die Tiefe (29) ist.

15. Milchpumpensystem (1) nach einem der vorhergehenden Ansprüche, wobei der Handgriff (11) ein in Längsrichtung (33) des Handgriffs (11) verschiebbares Anschlagelement (34) aufweist, das in zumindest einer Schiebestellung beim Verschwenken des Handgriffs (11) zur Betätigung des Pumpelements (10) am Verbindungselement (7) anschlägt, sodass das Anschlagelement (34) in der zumindest einen Schiebestellung einen Schwenkwinkel (35) des Handgriffs (11) begrenzt.
